# EUROPÄISCHE PATENTSCHRIFT

(11) **EP 1 440 102 B1**
(45) Veröffentlichungstag und Bekanntmachung des Hinweises auf die Patenterteilung: **18.02.2009**
(21) Anmeldenummer: 02797662.0
(22) Anmeldetag: 02.09.2002
(51) Int. Cl.: C08G 18/48, C07D 301/12, B01J 27/26, C08G 65/26

(54) **INTEGRIERTES VERFAHREN ZUR HERSTELLUNG VON POLYURETHAN-SCHÄUMEN**
INTEGRATED METHOD FOR THE PRODUCTION OF POLYURETHANE FOAMS
PROCEDE INTEGRE DE PRODUCTION DE MOUSSES DE POLYURETHANNE

(30) Priorität: 04.09.2001 DE 10143195
(43) Veröffentlichungstag der Anmeldung: 28.07.2004
(73) Patentinhaber: BASF SE, 67056 Ludwigshafen (DE)
(72) Erfinder: MÜLLER, Ulrich, 67435 Neustadt (DE); TELES, Joaquim, Henrique, 67166 Otterstadt (DE); BOHRES, Edward, 68161 Mannheim (DE); BAUER, Stephan, 49179 Ostercappeln (DE); RUPPEL, Raimund, 01127 Dresden (DE)
(74) Vertreter: Isenbruck, Günter
(86) Internationale Anmeldenummer: PCT/EP2002/009781
(87) Internationale Veröffentlichungsnummer: WO 2003/020787

(56) Entgegenhaltungen:
- EP-A- 1 022 300
- WO-A-99/51661
- US-A- 5 599 956

## Beschreibung

Die vorliegende Erfindung betrifft ein integriertes Verfahren zur Herstellung von Polyurethanen aus Isocyanaten und Polyetherpolyolen und/oder modifizierten Polyetherolen, die unter Verwendung von Propylenoxid erhältlich sind, insbesondere durch die Verwendung von Multimetallcyanid-Verbindungen als Katalysator, wobei das Propylenoxid durch Epoxidation von Propen mit mindestens einem Hydroperoxid hergestellt wird. Darüber hinaus betrifft die vorliegende Erfindung Polyurethane, die nach einem erfindungsgemäßen Verfahren herstellbar sind, sowie Formkörper, die ein erfindungsgemäß hergestelltes Polyurethan enthalten.

Erfindungsgemäß hergestellte Polyurethane eignen sich insbesondere zur Herstellung von Polyurethan-Schaumstoffen, Polyurethan Gießhäuten und Elastomeren.

Die Eigenschaften des Polyurethans, wie mechanische Eigenschaften, Geruch, hängen insbesondere stark von den zur Herstellung verwendeten Isocyanaten und Polyetheralkoholen und gegebenenfalls von den verwendeten Treibmitteln ab. Insbesondere die Struktur der Polyetheralkohole hat einen großen Einfluss auf die Eigenschaften der erhaltenen Polyurethane. Die Eigenschaften der Polyetheralkohole werden dabei durch das Herstellverfahren der Polyetheralkohole und insbesondere durch die Eigenschaften und das Herstellverfahren der Edukte beeinflusst.

Die Reduktion der Verunreinigungen in dem Propylenoxid und/oder Herstellung der Polyetheralkohole und/oder Polyurethane ist von vielfältigem Interesse. Von der Automobil- und Möbelindustrie werden zunehmend Polyurethane verlangt, die möglichst frei von Geruchsstoffen und Emissionen sind. So schreibt zum Beispiel die Prüfvorschrift von DaimlerChrysler PB VWL 709 vom 11.01.2001 verbindlich vor, dass Fahrzeuginnenteile einen maximalen Emissionswert für flüchtige Substanzen von 100 ppm und für kondensierbare Substanzen von 250 ppm aufweisen.

Zum einem führen die Verunreinigungen in den Polyurethanen in vielen Fällen zu geruchsintensiven Verbindungen. Dadurch sind die Polyurethane oder Polyurethan-Schaumstoffe nur eingeschränkt einsetzbar. Zum anderen führen die Verunreinigungen zu monofunktionellen Nebenverbindungen, wie insbesondere Allylalkohole, die die Funktionalität der Polyole gegenüber der theoretischen Starterfunktionalität herabsetzen und damit die mechanischen Eigenschaften signifikant verschlechtern, insbesondere beispielsweise Zugfestigkeit, Dehnung, Weiterreißfestigkeit, Härte und Abriebsfestigkeit.

Propylenoxid, hergestellt nach bekannten Verfahren zur Herstellung, die beispielsweise in Weissermel, Arpe "Industrielle Organische Chemie", VCH-Verlag, Weinheim, 4. Auflage, Seiten 288 bis 318 beschrieben sind, weist den Nachteil auf, dass deutliche Verunreinigungen im Propylenoxid enthalten sind. Die Verunreinigung liegen in einem Bereich von 5 bis 100 ppm.

Polyetheralkohole können beispielsweise durch basen- oder säurekatalysierte Polyaddition von Alkylenoxiden an polyfunktionelle Starterverbindungen hergestellt werden. Als Starterverbindungen sind beispielsweise Wasser, Alkohole, Säuren oder Amine oder Gemischen aus zwei oder mehr davon geeignet. Der Nachteil derartiger Herstellungsverfahren liegt insbesondere darin, dass aufwendige Reinigungsschritte nötig sind, um die Katalysatorreste vom Reaktionsprodukt abzutrennen. Darüber hinaus steigt bei derartig hergestellten Polyetherpolyolen mit zunehmender Kettenlänge der Gehalt an monofunktionellem Produkt und geruchsintensiven Verbindungen, die für die Polyurethanherstellung nicht erwünscht sind.

Die Verringerung der Funktionalität ist insbesondere für Elastomere von Nachteil, da die verwendeten Polyetheralkohole in der Regel bifunktionell sind. Durch die monofunktionellen Verunreinigungen im Polyetheralkohol wird die Funktionalität kleiner als 2, was eine signifikante Verschlechterung der mechanischen Eigenschaften des Polyurethans, insbesondere Zugfestigkeit und Dehnung bewirkt

Die durch die Nebenreaktionen der basen- oder säurekatalysierten Umsetzung entstehenden Nebenverbindungen sind darüber hinaus zum Teil als Geruchsträger im Polyurethan enthalten. Zu nennen sind beispielsweise Aldehyde, insbesondere Propionaldehyd, Cycloacetale, Allylalkohole und deren Umsetzungsprodukte. Von der Auto- und Möbelindustrie werden zunehmend geruchsarme oder geruchslose Polyetherole und Polyurethane gefordert.

Multimetallcyanid-Verbindungen sind aus dem Stand der Technik als Katalysatoren für Polyadditionen, insbesondere für Ring-öffnende Polymerisationen von Alkylenoxiden bekannt, wie beispielsweise in der EP-A 0 892 002, EP-1 0 862 977 und in der EP-A 0 755 716 beschrieben.

Die WO 01/16209 beschreibt ein Verfahren zur Herstellung von Polyetheralkoholen durch katalysierte Anlagerung von Ethylenoxid und Propylenoxid an H-funktionelle Starterverbindungen in Gegenwart einer Multimetallcyanidverbindung.

So beschreibt beispielsweise die WO 00/78837 die Verwendung von mittels Multimetallcyanid-Katalysatoren aus Propylenoxid hergestellten Polyetherpolyolen zur Herstellung von Polyurethan-Weichschäumen. Problematisch ist hierbei jedoch, dass bereits geringe Mengen von Verunreinigungen im Propylenoxid zur Belegung des Multimetallcyanid-Katalysators führen und damit die Aktivität des Katalysators senken. Darüber hinaus können Verunreinigungen im Polyetherpolyol, die bereits im Propylenoxid vorlagen, zu einer Verunreinigung des hergestellten Polyurethans führen. In diesem Zusammenhang sind insbesondere niedermolekulare Verbindungen zu nennen, die zu einer Geruchsbelästigung führen. Derartige Verunreinigungen können aus den Propylenoxid oder den daraus hergestellten polymeren Produkten nur durch aufwendige Reinigungsschritte entfernt werden. Als Verunreinigungen sind insbesondere Aldehyde und Ketone zu nennen.

Eine Aufgabe der vorliegenden Erfindung lag daher darin, ein Verfahren zur Herstellung von Polyurethanen bereitzustellen, das ohne aufwendige Reinigungsschritte der Edukte und Zwischenprodukte Polyurethane liefert, die arm an Verunreinigungen, insbesondere an niedermolekularen geruchsintensiven Verbindungen, sind.

Erfindungsgemäß wird diese Aufgabe gelöst durch ein integriertes Verfahren zur Herstellung eines Polyurethans aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxid zu einem Polyetheralkohol, unter Verwendung mindestens einer Multimetallcyanid-Verbindung als Katalysator;
(3) Umsetzung eines Polyetheralkohols aus Schritt (2) mit mindestens einem Isocyanat.

Unter Polyetheralkoholen werden im Rahmen der vorliegenden Erfindung insbesondere Polyetherpolyole und modifizierte Polyetherole verstanden, die unter Verwendung von Propylenoxid erhältlich sind.

Als besonders geeignetes Hydroperoxid für die Epoxidation gemäß Schritt (1) hat sich im Rahmen der vorliegenden Erfindung Wasserstoffperoxid erwiesen. Dieses kann außerhalb der Umsetzung gemäß (1) aus Wasserstoff und Sauerstoff oder in situ in der Umsetzung gemäß (1) hergestellt werden.

Daher betrifft die vorliegende Erfindung in einer bevorzugten Ausführungsform ein integriertes Verfahren zur Herstellung eines Polyurethans, wobei das in Schritt (1) eingesetzte Hydroperoxid Wasserstoffperoxid ist.

Die Epoxidation gemäß Schritt (1) ist prinzipiell beispielsweise aus der DE 10055652.3 und weiteren Patentanmeldungen der Anmelderin wie z. B. DE 10032885.7, DE 10032884.9, DE 10015246.5, DE 19936547.4, DE 19926725.1, DE 19847629.9, DE 19835907.1, DE 19723950.1, bekannt. Mittels der Epoxidation gemäß Schritt (1) wird Propylenoxid in einer hohen Reinheit erhalten. So weist das Propylenoxid insbesondere einen Gehalt an C6-Verbindungen von < 1 ppm auf.

Unter C6-Verbindungen werden im Rahmen der vorliegenden Erfindung beispielsweise 2-Methylpentan, 4-Methylpenten-1, n-Hexan, Hexene wie 1-Hexen, sowie Komponenten mit 6 C-Atomen und zusätzlich einer oder mehrerer funktionellen Gruppen aus der Klasse der Aldehyde, Carbonsäuren, Alkohole, Ketone, Ether verstanden. Weitere unerwünschte Verunreinigungen sind Propanderivate, insbesondere chlorierte Propanderivate, Acetaldehyd, Propionaldehyd, Aceton, Dioxolane, Allylalkohol, Pentan, Methylpentane, Furan, Hexan, Hexen, Methoxypropan oder auch Methanol.

Als weitere Nebenkomponenten kann das in Schritt (1) erhaltene Propylenoxid auch noch bis zu 100 ppm, insbesondere bis zu 40 ppm Methanol und bis zu 10 ppm, bevorzugt bis zu 4 ppm Acetaldehyd enthalten.

Gegenüber anderen bekannten Verfahren zur Herstellung von Propylenoxid, die beispielsweise in Weissermel, Arpe "Industrielle Organische Chemie", VCH-Verlag, Weinheim, 4. Auflage, Seiten 288 bis 318 beschrieben sind, wird mittels des erfindungsgemäßen Schritt (1) Propylenoxid erhalten, das sehr geringe Verunreinigungen an C6-Komponenten und keine chlororganischen Verunreinigungen enthält.

In einer weiteren Ausführungsform betrifft die vorliegende Erfindung daher ein integriertes Verfahren zur Herstellung eines Polyurethans, wobei das in Schritt (1) erhaltene Propylenoxid einen Gehalt an C6-Verunreinigungen < 1 ppm aufweist.

Geeignete Bedingungen für die Epoxidation gemäß Schritt (1) sind beispielsweise in der DE 100 55 652.3 beschrieben.

Die Umsetzung des Propens mit einem Hydroperoxid, insbesondere Wasserstoffperoxid, findet im Rahmen des erfindungsgemäßen Verfahrens bevorzugt in Gegenwart eines Katalysators statt. Als Katalysatoren zur Umsetzung des Propylens zu Propylenoxid sind prinzipiell alle, bevorzugt alle heterogenen Katalysatoren denkbar, die für die jeweilige Umsetzung geeignet sind.

Bevorzugt werden dabei Katalysatoren verwendet, die ein poröses oxidisches Material wie z.B. einen Zeolith umfassen. Vorzugsweise werden Katalysatoren eingesetzt, die als poröses oxidisches Material ein Titan-, Vanadium-, Chrom-, Niob-, Zinn-, Germanium- oder Zirkonium-haltigen Zeolith umfassen.

Im besonderen existieren Zeolithe, die kein Aluminium enthalten und bei denen im Silikatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978.

Titanzeolithe mit MFI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Vanadium-, Chrom-, Niob-, Zinn-, Germanium-, Zirkonium enthaltende Zeolithe mit Pentasil-Zeolith-Struktur zu nennen, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WEI-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-12 zu nennen.

Als besonders bevorzugt sind für das erfindungsgemäße Verfahren Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu beta-Zeolith isomorphen Gerüststruktur zu nennen.

Insbesondere bevorzugt wird im erfindungsgemäßen Verfahren ein heterogener Katalysator, der das titanhaltige Silikalit TS-1 umfasst, verwendet.

In einer weiteren Ausführungsform betrifft die vorliegende Erfindung daher eine integriertes Verfahren zur Herstellung eines Polyurethans, wobei die Epoxidation gemäß Schritt (1) in Gegenwart eines Zeolithkatalysators, insbesondere eines titanhaltigen Zeolithkatalysators, durchgeführt wird.

Das durch die Epoxidation gemäß Schritt (1) erhaltene Propylenoxid wird erfindungsgemäß in Gegenwart von geeigeneten Katalysatoren zu einem Polyetheralkohol umgesetzt. Als Katalysatoren werden heterogene Katalysatoren nämlich Multimetallcyanidkatalysatoren verwendet.

Üblicherweise wird nach der Synthese der Katalysator durch Neutralisation, Destillation und Filtration entfernt. Bei der Multimetallcyanid-Katalyse wird der Katalysator abfiltriert, durch Filtration abgereichert und/oder verbleibt im Polyetherol.

Erfindungsgemäß wird die Umsetzung zu Polyetheralkoholen in Gegenwart von Multimetallcyanidkatalysatoren durchgeführt.

Bei der Umsetzung zu Polyetheralkoholen kann das gemäß Schritt (1) erhaltene Propylenoxid direkt in die Umsetzung gemäß Schritt (2) eingesetzt werden. Es ist jedoch im Rahmen der vorliegenden Erfindung ebenso möglich, dass das Propylenoxid aus Schritt (1) zunächst behandelt wird, beispielsweise gereinigt wird. Als Reinigung kommt erfindungsgemäß beispielsweise Feindestillation in Frage. Geeignete Verfahren werden beispielsweise in der EP-B 0 557 116 beschrieben.

Das gemäß Schritt (1) erhaltene Propylenoxid kann im Rahmen der vorliegenden Erfindung alleine oder zusammen mit mindestens einem weiteren Alkylenoxid eingesetzt werden. Im Rahmen der vorliegenden Erfindung können zur Herstellung eines Polyetheralkohols gemäß Schritt (2) neben dem gemäß Schritt (1) erhaltenen Propylenoxid prinzipiell alle Alkylenoxide eingesetzt werden, die dem Fachmann bekannt sind. Insbesondere werden substituierte oder unsubstituierte Alkylenoxide mit 2 bis 24 C-Atomen, beispielsweise Alkylenoxide mit Halogen-, Hydroxy-, nicht cyclische Ether- oder Ammoniumsubstituenten eingesetzt.

Erfindungsgemäß geeignet sind beispielsweise: Ethylenoxid, 1,2-Epoxypropan, 1,2-Epoxy-2-methylpropan, 1,2-Epoxybutan, 2,3-Epoxybutan, 1,2-Epoxy-3-methylbutan, 1,2-Epoxypentan, 1,2-Epoxy-3-methylpentan, 1,2-Epoxyhexan, 1,2-Epoxyheptan, 1,2-Epoxyoctan, 1,2-Epoxynonan, 1,2-Epoxydecan, 1,2-Epoxyundecan, 1,2-Epoxydodecan, 1,2-Epoxycyclopentan, 1,2-Epoxycyclohexan, (2,3-Epoxypropyl)benzol, Vinyloxiran, 3-Phenoxy-1,2-epoxgpropan> 2,3-Epoxymethylether, 2,3-Epoxyethylether, 2,3-Epoxyisopropylether, 2,3-Epoxy-1-propanol, (3,4-Epoxybutyl)stearat, 4,5-Epoxypentylacetat, 2,3-Epoxypropanmethacrylat, 2,3-Epoxypropanacrylat, Glycidylbutyrat, Methylglycidat, Ethyl-2,3-epoxybutanoat, 4-(Trimethylsilyl)butan-1,2-epoxid, 4-(Triethylsilyl)butan-1,2-epoxid, 3-(Perffuoromethyl)propenoxid, 3-(Perfluoroethyl)propenoxid, 3-(Perfluorobutyl)propenoxid 4-(2,3-Epoxypropyl)morpholin, 1-(Oxiran-2-ylmethyl)pyrrolidin-2-on, sowie Gemische aus zwei oder mehr davon.

Insbesondere sind zu nennen: aliphatische 1,2-Alkylenoxide mit 2 bis 4 C-Atomen, beispielsweise Ethylenoxid, 1,2-Butylenoxid, 2,3-Butylenoxid oder Isobutylenoxid, aliphatische 1,2-Alkylenoxide mit 5 bis 24 C-Atomen, cycloaliphatische Alkylenoxide, beispielsweise Cyclopentenoxid, Cyclohexenoxid oder Cyclododecatrien-(1,5,9)-monoxid, araliphatische Alkylenoxide, beispielsweise Styroloxid.

Besonders bevorzugt sind im Rahmen der vorliegenden Erfindung Ethylenoxid, 1,2-Epoxypropan, 1,2-Epoxybutan, 2,3-Epoxybutan, Styroloxid, Vinyloxiran und deren beliebige Mischungen untereinander, insbesondere Ethylenoxid, 1,2-Epoxypropan und Mischungen aus Ethylenoxid und 1,2-Epoxypropan.

Sofern neben dem gemäß Schritt (1) erhaltenen Propylenoxid mindestens ein weiteres Alkylenoxid eingesetzt wird, ist es erfindungsgemäß möglich, dass ein Gemisch aus dem gemäß Schritt (1) erhaltenen Propylenoxid und mindestens einem weiteren Alkylenoxid eingesetzt wird. Ebenso ist es im Rahmen der vorliegenden Erfindung jedoch möglich, dass das gemäß Schritt (1) erhaltene Propylenoxid und mindestens ein weiteres Alkylenoxid nacheinander zugesetzt werden.

Die durch die Umsetzung gemäß Schritt (2) erhaltenen Polyetheralkohole können beispielsweise auch Blockstrukturen aufweisen. Die Struktur der Polyetheralkohole kann dabei durch geeignete Reaktionsbedingungen in weiten Bereichen gesteuert werden. Geeignete Reaktionsbedingungen für die Umsetzung gemäß Schritt (2) sind beispielsweise in der WO 99/16775 beschrieben.

Die gemäß Schritt (2) erhaltenen Polyetheralkoholen können für die Umsetzung gemäß Schritt (3) gegebenenfalls modifiziert werden. Als modifizierte Polyetherole sind insbesondere zu nennen Pfropfpolyetherpolyole, insbesondere solche, die durch Polymerisation von Styrol und Acrylnitril in Gegenwart von Polyetherolen hergestellt werden, Polyharnstoffdispersionen (PHD-Polyole), die durch Umsetzung von Diisocyanaten und Diaminen in Gegenwart von Polyetherolen hergestellt werden und Polyisocyanat-Polyadditions-Polyole (PIPA-Polyole), die durch Umsetzung von Diisocyanaten und Aminoalkoholen in Gegenwart von Polyetherolen hergestellt werden, zu nennen.

Die Umsetzung gemäß Schritt (2) erfolgt in Gegenwart einer Multimetallcyanidverbindung als Katalysator. Geeignete Katalysatoren sind beispielsweise in der WO 99/16775 und der DE 10117273.7 beschrieben. Erfindungsgemäß werden insbesondere Multimetallcyanidverbindung der allgemeinen Formel I als Katalysator für die Umsetzung gemäß Schritt (2) eingesetzt:

M¹ₐ[M²(CN)_{b}(A)_{c}]_{d} · fM¹_{g}Xₙ·h(H₂O)·eL·kP (I),

in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr^{z+}, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle und eine gebrochenen oder ganze Zahl größer 0 oder 0 ist,
- f, k, h und m unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Multimetallcyanidverbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Multimetallcyanidverbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Multimetallcyanid-Katalysatoren bevorzugt. Ferner bevorzugt sind Multimetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Multimetallcyanid-Phase hergestellt und diese anschließend durch Umkristallisation in eine aktive Multimetallcyanidphase überführt, wie in der PCT/EPO1/01893 beschrieben.

Bei einer anderen bevorzugten Ausführungsform der Katalysatoren sind f, e und k ungleich Null. Dabei handelt es sich um Multimetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten (WO 98/06312). Die Katalysatoren können entweder unter starkem Rühren (24000U/Min mit Turrax) oder unter Rühren hergestellt werden (US 5,158,922).

Ebenfalls geeignete Katalysatoren werden in der Anmeldung WO 01/03830 beschrieben. Derartige Multimetallcyanid-Katalysatoren werden mit organischen Sulfonen der allgemeinen Form R-S(O)₂-R oder Sulfoxiden der allgemeinen Form R-S(O)-R als organischem komplexierenden Agens hergestellt. Als Vorteile des Katalysators sind kurze Induktionszeiten und moderate Exothermie zu nennen. In WO 01/03831 wird eine weitere Variante der Katalysatorsynthese beschrieben. Dabei werden Multimetallcyanid-Katalysatoren durch eine "Incipient Wetness Method" synthetisiert. Diese Katalysatoren können ebenfalls für das erfindungsgemäße Verfahren eingesetzt werden.

Weitere erfindungsgemäß geeignete Multimetallcyanid-Katalysatoren aus Metal[hexacyanometallat-hexanitrometallat] werden in der Anmeldung WO 01/04182 erwähnt. Die dort genannten Ausgangsverbindungen sind kostengünstiger, als die in der Regel verwendete Zinkhexacyanocobaltate. Außerdem zeigen die Katalysatoren kürzere Induktionszeiten und in manchen Fällen zeigen sie eine moderate Exothermie. Die so hergestellte Multimetallcyanid-Katalysatoren können aber auch geträgert werden, wie in den Anmeldungen WO 01/04180 (Polycarbonsäuren) und WO 01/04177 (Zeolithe) beschrieben wird. Dadurch kann eine einfache Abtrennung des Katalysators erreicht werden.

Ein ebenfalls erfindungsgemäß geeigneter Multimetallcyanid-Katalysator kann gemäß der WO 01/04181 auf Basis von Hexacyanocobaltat-Nitroferrocyanid hergestellt werden. Diese Katalysatoren zeigen kurze Induktionszeiten bei der Polymerisation von Alkylenoxide zu Polyethern.

Insbesondere als Katalysator geeignet sind für das erfindungsgemäße Verfahren Multimetallcyanid-Verbindungen, die Zink, Kobalt oder Eisen oder zwei davon enthalten. Besonders geeignet ist beispielsweise Berliner Blau.

Daher betrifft die vorliegende Erfindung in einer bevorzugten Ausführungsform ein integriertes Verfahren zur Herstellung eines Polyurethans, wobei die Multimetallcyanidverbindung Zink, Kobalt oder Eisen oder zwei davon enthält.

Erfindungsgemäß wird gemäß Schritt (2) Propylenoxid aus Schritt (1) oder ein Gemisch aus dem Propylenoxid aus Schritt (1) und mindestens einem weiteren Alkylenoxid mit einer Starterverbindung umgesetzt.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 4,4'-, 2,4'- und 2,2'-Diamino-di-phenylmethan. Als Startermoleküle kommen ferner in Betracht: Alkanolamine, wie z. B. Ethanolamin, N-Methyl- und N-Ethyl-ethanolamin, Dialkanolamine, wie z. B. Diethanolamin, N-Methyl- und N-Ethyl-diethanolamin, und Trialkanolamine, wie z. B. Triethanolamin, und Ammoniak sowie mehrwertige Alkohole, wie Monoethylenglykol, Propandiol-1,2 und-1,3, Diethylenglykol, Dipropylenglykol, Butandiol-1,4, Hexandiol-1,6, Glycerin, Trimethylolpropan, Pentaerythrit, Sorbit und Saccharose. Bevorzugt werden als Polyetherpolyalkohole Anlagerungsprodukte von Ethylenoxid und/oder Propylenoxid an Wasser, Monoethylenglykol, Diethylenglykol, Propandiol-1,2, Diproplyenglykol Glycerin, Trimethylolpropan, Ethylendiamin, Triethanolamin, Pentaerythrit, Sorbit und/oder Saccharose einzeln oder in Mischungen eingesetzt.

Die Startersubstanzen können erfindungsgemäß auch in Form von Alkoxylaten, insbesondere solche mit einem Molekulargewicht Mw in Bereich von 62 bis 15000 g /Mol zum Einsatz kommen.

Ebenso geeignet sind jedoch auch Makromoleküle mit funktionellen Gruppen, die aktive Wasserstoff-Atome aufweisen, beispielsweise Hydroxylgruppen, insbesondere solche, die in der WO 01/16209 genannt sind.

Die gemäß Schritt (2) erhaltenen Polyetheralkohole können gemäß Schritt (3) mit. Isocyanaten umgesetzt werden. Dabei kann Schritt (3) direkt an Schritt (2) anschließen. Es ist jedoch ebenso möglich, dass ein zusätzlicher Schritt, insbesondere ein Reinigungsschritt, zwischen Schritt (2) und Schritt (3) durchgeführt wird.

Im Rahmen der vorliegenden Erfindung können ein oder mehrere Isocyanate eingesetzt werden. Neben dem Polyetheralkohol, der aus Schritt (2) erhalten wird, können für die Umsetzung gemäß Schritt (3) auch noch weitere Verbindungen mit gegenüber Isocyanaten reaktiven Gruppen, insbesondere mit Hydroxylgruppen, eingesetzt werden.

Als weitere OH-Komponenten können beispielsweise Polyester, weitere Polyether, Polyacetale, Polycarbonate, Polyesterether und dergleichen eingesetzt werden.

Geeignete Polyesterpolyole können beispielsweise aus organischen Dicarbonsäuren mit 2 bis 12 Kohlenstoffatomen, vorzugsweise aliphatischen Dicarbonsäuren mit 4 bis 6 Kohlenstoffatomen, und mehrwertigen Alkoholen, vorzugsweise Diolen, mit 2 bis 12 Kohlenstoffatomen, vorzugsweise 2 bis 6 Kohlenstoffatomen hergestellt werden. Als Dicarbonsäuren kommen beispielsweise in Betracht: Bernsteinsäure, Glutarsäure, Adipinsäure, Korksäure, Azelainsäure, Sebacinsäure, Decandicarbonsäure, Maleinsäure, Fumarsäure, Phthalsäure, Isophthalsäure und Terephthalsäure. Die Dicarbonsäuren können dabei sowohl einzeln als auch im Gemisch untereinander verwendet werden. Anstelle der freien Dicarbonsäuren können auch die entsprechenden Dicarbonsäurederivate, wie z. B. Dicarbonsäureester von Alkoholen mit 1 bis 4 Kohlenstoffatomen oder Dicarbonsäureanhydride eingesetzt werden. Beispiele für zwei- und mehrwertige Alkohole sind: Ethandiol, Diethylenglykol, 1,2- bzw. 1,3-Propandiol, Dipropylenglykol, 1,4-Butandiol, 1,5-Pentandiol, 1,6-Hexandiol, 1,10-Decandiol, 1,12-Dodecandiol, Glycerin und/oder Trimethylolpropan. Vorzugsweise verwendet werden Ethandiol, Diethylenglykol, 1,4-Butandiol, 1,5-Pentandiol, 1,6-Hexandiol, Glycerin und/oder Trimethylolpropan. Eingesetzt werden können ferner Polyesterpolyole aus Lactonen, z. B. Caprolacton oder Hydroxycarbonsäuren, z.B. Hydroxycapronsäure. Zur Herstellung der Polyesterpolyole können die organischen, z. B. aromatischen und vorzugsweise aliphatischen, Polycarbonsäuren und/oder -derivate und mehrwertigen Alkohole katalysatorfrei oder vorzugsweise in Gegenwart von Veresterungskatalysatoren, zweckmäßigerweise in einer Atmosphäre aus Inertgas, wie z. B. Stickstoff, Kohlenmonoxid, Helium, Argon u. a., in der Schmelze bei Temperaturen von 150 bis 250°C, vorzugsweise 180 bis 220°C gegebenenfalls unter vermindertem Druck bis zu der gewünschten Säurezahl, die vorteilhafterweise kleiner als 10, vorzugsweise kleiner als 2 ist, polykondensiert werden. Nach einer bevorzugten Ausführungsform wird das Veresterungsgemisch bei den obengenannten Temperaturen bis zu einer Säurezahl von 80 bis 30, vorzugsweise 40 bis 30, unter Normaldruck und anschließend unter einem Druck von kleiner als 500 mbar, vorzugsweise 50 bis 150 mbar, polykondensiert. Als Veresterungskatalysatoren kommen beispielsweise Eisen-, Cadmium-, Kobalt-, Blei-, Zink-, Antimon-, Magnesium-, Titan- und Zinnkatalysatoren in Form von Metallen, Metalloxiden oder Metallsalzen in Betracht. Die Polykondensation kann jedoch auch in flüssiger Phase in Gegenwart von Verdünnungs- und/oder Schleppmitteln, wie z. B. Benzol, Toluol, Xylol oder Chlorbenzol, zur azeotropen Abdestillation des Kondensationswassers durchgeführt werden. Zur Herstellung der Polyesterpolyole werden die organischen Polycarbonsäuren und/oder -derivate und mehrwertigen Alkohole vorteilhafterweise im Molverhältnis von 1 : 1 bis 1,8, vorzugsweise 1 : 1,05 bis 1,2, polykondensiert. Die erhaltenen Polyesterpolyole besitzen vorzugsweise eine Funktionalität von 2 bis 4, insbesondere 2 bis 3, und eine Hydroxylzahl von bevorzugt 20 bis 200 mgKOH/g. Des weiteren können als gegenüber Isocyanaten reaktive Verbindungen Diole, Triole und/oder Polyole mit Molekulargewichten von 60 bis <400 eingesetzt werden, beispielsweise aliphatische, cycloaliphatische und/oder araliphatische Diole mit 2 bis 14, vorzugsweise 4 bis 10 Kohlenstoffatomen, wie z. B. Ethylenglykol, Propandiol-1,3, Decandiol-10, o-, m-, p-Dihydroxycyclohexan, Diethylenglykol, Dipropylenglykol und vorzugsweise Butandiol-1,4, Hexandiol-1,6 und Bis-(2-hydroxyethyl)-hydrochinon, Triole, wie 1,2,4-, 1,3,5-Trihydroxy-cyclohexan, Glycerin und Trimethylol-Propan und niedermolekulare Hydroxylgruppen haltige Polyalkylenoxide auf Basis Ethylen- und/oder 1,2-Propylenoxid und den vorgenannten Diolen und/oder Triolen als Startermoleküle.

Erfindungsgemäß wird der Polyetheralkohol aus Schritt (2) mit mindestens einem Isocyanat umgesetzt. Prinzipiell sind erfindungsgemäß alle dem Fachmann bekannten Isocyanate geeignet. Insbesondere sind zu nennen: aromatische, araliphatische, aliphatische und/oder cycloaliphatische organische Isocyanate, bevorzugt Diisocyanate. Im einzelnen seien beispielhaft genannt: Alkylendiisocyanate mit 4 bis 12 Kohlenstoffatomen im Alkylenrest, wie 1,12-Dodecandiisocyanat, 2-Ethyltetramethylendiisocyanat-1,4, 2- Methylpentamethylendüsocyanat-1,5, Tetramethylendiisocyanat-1,4, Lysinesterdiisocyanate (LDI) und/oder Hexamethylendiisocyanat-1,6 (HDI); cycloaliphatische Diisocyanate wie Cyclohexan-1,3- und 1,4-diisocyanat sowie beliebige Gemische dieser Isomeren, 2,4- und 2,6-Hexahydrotoluylendiisocyanat sowie die entsprechenden Isomerengemische, 4,4'-, 2,2'- und 2,4'-Dicyclohcxylmethandiisocyanat sowie die entsprechenden Isomerengemische und/oder 1-lsocyanato-3,3,5-trimethyl-5-isocyanatomethylcyclohexan (IPDI). Des weiteren sind als Isocyanate beispielhaft zu nennen: 2,4- und 2,6-Toluylendiisocyanat und die entsprechenden Isomerengemische, 4,4'-, 2,4'- und 2,2'-Diphenylmethandiisocyanat und die entsprechenden Isomerengemische, Mischungen aus 4,4'- und 2,2'-Diphenylmethandiisocyanaten, Polyphenylpolymethylenpolyisocyanate, Mischungen aus 4,4'-, 2,4'- und 2,2'-Diphenylinethandiisocyanaten und Polyphenylpolymethylen-polyisocyanaten (Roh-MDI) und Mischungen aus Roh-MDl und Toluylendiisocyanaten. Außerdem können Mischungen enthaltend mindestens zwei der genannten Isocyanate eingesetzt werden. Des weiteren können modifizierte Isocyanate, d.h. Isocyanurat-, Biuret-, Ester-, Harnstoff-, Allophanat-, Carbodiimid-, Uretdion- und/oder Urethangruppen, letztere im Folgenden auch als Urethangruppen-modifiziert bezeichnet, enthaltende Di- und/oder Polyisocyanate in dem erfindungsgemäßen Verfahren eingesetzt werden. Im einzelnen kommen beispielsweise in Betracht: Urethangruppen enthaltende organische Polyisocyanate mit NCO-Gehalten von 50 bis 10 Gew.-%, vorzugsweise von 35 bis 15 Gew.-%, bezogen auf das Gesamtgewicht, beispielsweise mit niedermolekularen Diolen, Triolen, Dialkylenglykolen, Trialkylenglykolen oder Polyoxyalkylenglykolen mit Molekulargewichten bis 6000, insbesondere mit Molekulargewichten bis 1500, modifiziertes 4,4'-Diphenylmethandiisocyanat, modifizierte 4,4'- und 2,4'-Diphenylmethandiisocyanatmischungen, modifiziertes Roh-MD1 oder 2,4- bzw. 2,6-Toluylendiisocyanat, wobei als Di- bzw. Polyoxyalkylenglykole, die einzeln oder als Gemische eingesetzt werden können, beispielsweise genannt seien: Diethylen-, Dipropylenglykol, Polyoxyethylen-, Polyoxypropylen- und Polyoxypropylenpolyoxyethenglykole, -triole und/oder -tetrole. Geeignet sind auch NCO-Gruppen enthaltende Prepolymere mit NCO-Gehalten von 25 bis 3,5 Gew.-%, vorzugsweise von 21 bis 14 Gew.-%, bezogen auf das Gesamtgewicht, hergestellt aus den beschriebenen Polyester- und/oder vorzugsweise Polyetherpolyolen und 4,4'-Diphenylmethandiisocyanat, Mischungen aus 2,4'- und 4,4'-Diphenylmethandiisocyanat, 2,4- und/oder 2,6-Toluylendiisocyanaten oder Roh-MD1. Bewährt haben sich ferner flüssige, Carbodiimidgruppen enthaltende Polyisocyanate mit NCO-Gehalten von 33,6 bis 15, vorzugsweise 31 bis 21 Gew.-%, bezogen auf das Gesamtgewicht, z. B. auf Basis von 4,4'-, 2,4'- und/oder 2,2'-Diphenylmethandiisocyanat und/oder 2,4- und/oder 2,6-Toluylendiisocyanat. Die modifizierten Polyisocyanate können miteinander oder mit unmodifizierten organischen Polyisocyanaten wie z. B. 2,4'-, 4,4'-Diphenylmethandiisocyanat, Roh-MD1, 2,4- und/oder 2,6-Toluylendiisocyanat gegebenenfalls gemischt werden. Als modifizierte Isocyanate setzt man bevorzugt isocyanuratisierte, biuretisierte und/oder Urethangruppen-modifizierte aliphatische und/oder cycloaliphatische Diisocyanate, beispielsweise die bereits genannten, ein, die nach allgemein bekannten Verfahren biuretisiert und/oder cyanuratisiert worden sein können und mindestens eine, bevorzugt mindestens zwei freie Isocyanatgruppen aufweisen, besonders bevorzugt drei freie Isocyanatgruppen aufweisen. Die Trimerisierung der Isocyanate zur Herstellung der Isocyanate mit Isocyanuratsruktur kann bei üblichen Temperaturen in Gegenwart bekannter Katalysatoren, beispielsweise Phosphinen und/oder Phosphinderivaten, Aminen, Alkalisalzen, Metallverbindungen und/oder Mannichbasen erfolgen. Trimerisierte Isocyanate enthaltend Isocyanuratstrukturen sind zudem kommerziell erhältlich. Isocyanate mit Biuretstrukturen können nachallgemein bekannten Verfahren beispielsweise durch Reaktion von den genannten Diisocyanaten mit Wasser oder beispielsweise Diaminen hergestellt werden, wobei als Zwischenprodukt ein Harnstoffderivat gebildet wird. Auch biuretisierte Isocyanate sind kommerziell erhältlich.

Die Umsetzung gemäß Schritt (3) erfolgt unter den dem Fachmann bekannten Bedingungen. Geeignete Reaktionsbedingungen sind beispielsweise in Becker, Braun "Polyurethane", Kunststoffhandbuch, Band 7, Carl Hanser Verlag, München, 3. Auflage, 1993, S. 139 bis 193 beschrieben.

Gegebenenfalls können bei der Umsetzung gemäß Schritt (3) noch weitere, niedermolekulare Verbindungen als Additive vorliegen. Solche Verbindungen können beispielsweise als Kettenverlängerer oder Abstoppreagenzien wirken. Hierzu geeignet sind beispielsweise primäre Aminoverbindungen mit zwei bis etwa 20, beispielsweise 2 bis etwa 12 C-Atomen. Beispielsweise sind dies Ethylamin, n-Propylamin, i-Propylamin, sek.-Propylamin, tert.-Butylamin, 1-Aminoisobutan, substituierte Amine mit zwei bis etwa 20 C-Atomen wie 2-(N,N-Dimethylamino)-1-Aminoethan, Aminomercaptane wie 1-Amino-2-mercaptoethan, Diamine, aliphatische Aminoalkohole mit 2 bis etwa 20, vorzugsweise 2 bis etwa 12 C-Atomen, beispielsweise Methanolamin, 1-Amino-3,3-dimethyl-pentan-5-ol, 2-Aminohexan-2',2"-diethanolamin, 1-Amino-2,5-dimethylcyclohexan-4-ol, 2-Aminopropanol, 2-Aminobutanol, 3-Aminopropanol, 1-Amino-2-propanol, 2-Amino-2-methyl-1-propanol, 5-Aminopentanol, 3-Aminomethyl-3,5,5-trimethylcyclohexanol, 1-Amino-1-cyclopentan-methanol, 2-Amino-2-ethyl-1,3-propandiol, aromatisch-aliphatische oder aromatisch-cycloaliphatische Aminoalkohole mit 6 bis etwa 20 C-Atomen, wobei als aromatische Strukturen heterocyclische Ringsysteme oder vorzugsweise isocyclische Ringsysteme wie Naphthalin- oder insbesondere Benzolderivate wie 2-Aminobenzylalkohol, 3-(Hydroxymethyl)anilin, 2-Amino-3-phenyl-1-propanol, 2-Amino-1-phenylethanol, 2-Phenylglycinol oder 2-Amino-1-phenyl-1,3-propandiol sowie Gemische aus zwei oder mehr solcher Verbindungen in Betracht kommen.

Die Umsetzung gemäß Schritt (3) kann gegebenenfalls in Gegenwart eines Katalysators vorgenommen werden. Als Katalysator geeignet sind prinzipiell alle Verbindungen, die die Reaktion von Isocyanaten mit den gegenüber Isocyanaten reaktiven Verbindungen stark beschleunigen, wobei vorzugsweise ein Gesamtkatalysatorgehalt von 0,001 bis 15 Gew.-%, insbesondere 0,05 bis 6 Gew.-%, bezogen auf das Gewicht der insgesamt eingesetzten gegenüber Isocyanaten reaktiven Verbindungen (b), verwendet wird. Im Folgenden seien mögliche Katalysatoren (c) beispielhaft genannt: tertiäre Amine, beispielsweise Triethylamin, Tributylamin, Dimethylbenzylamin, Dicyclohexylmethylamin, Dimethylcyclohexylamin, N,N,N',N'-Tetramethyl-diamino-diethylether, Bis-(dimethylaminopropyl)-harnstoff, N-Methyl- bzw. N-Ethylmorpholin, N-Cyclohexylmorpholin, N,N,N',N'-Tetramethylethylendiamin, N,N,N',N'- Tetramethylbutandiamin, N,N,N',N'-Tetramethylhexandiamin-1,6, Pentamethyldiethylentriamin, Dimethylpiperazin, N-Dimethylaminoethylpiperidin, 1,8-Diazabicyclo(5.4.0)undecen-7, 1,2-Dimethylimidazol, 1-Azabicyclo-(2,2,0)-octan, 1,4-Diazabicyclo-(2,2,2)-octan (DABCO), Alkanolaminverbindungen, wie Triethanolamim, Triisopropanolamin, N-Methyl- und N-Ethyl-diethanolamin, Dimethylaminoethanol, 2-(N,N-Di-methylaminoethoxy)ethanol, N,N',N"-Tris-(dialkylaminoalkyl)hexahydrotriazine, z. B. N,N',N"-Tris-(dimethylaminopropyl)-s-hexahydrotriazin, bevorzugt Triethylendiamin, Pentamethylendiethylentriamin und/oder Bis(dimethylamino)ether; Metallsalze, beispielsweise anorganische und/oder organische Verbindungen des Eisens, Bleis, Zinks, und/oder Zinns in üblichen Oxidationsstufen des Metalls, beispielsweise Eisen(II)-chlorid, Zinkchlorid, Bleioctoat und vorzugsweise Zinnverbindungen, wie Zinn(II)-Verbindungen, insbesondere Zinndioctoat, Zinndiethylhexanoat und/oder Zinn(IV)-Verbiundungen, wie Di-alkyl-zinn-di(isooctylmercaptoacetat), Di-alkyl-zinn-di(2-ethyl-hexylmaleat), Di-alkyl-zinn-di(2-ethyl-hexylmercaptoacetat), Di-alkyl-zinn-di(isooctylmercaptoacetat), Di-alkyl-zinn-dilaurat, Di-alkyl-zinndimaleat, Di-alkyl-zinn-di(mercaptoacetat); Weiterhin können Amidine, wie 2,3-Dimethyl-3,4,5,6-tetrahydropyrimidin, Tetraalkylammonium-hydroxide, wie Tetramethylammoniumhydroxid, Alkalihydroxide, wie Natriumhydroxid und Alkalialkoholate, wie Natriummethylat und Kaliumisopropylat, sowie Alkalisalze von langkettigen Fettsäuren mit 10 bis 20 C-Atomen und gegebenenfalls seitenständigen OH-Gruppen als Katalysatoren verwendet werden. Die beispielhaft genannten Katalysatoren können einzeln oder in Mischungen enthaltend mindestens zwei der genannten Katalysatoren eingesetzt werden.

Als Hilfs- und/oder Zusatzstoffe können gegebenenfalls übliche Substanzen in dem erfindungsgemäßen Verfahren verwendet werden. Genannt seien beispielsweise oberflächenaktive Substanzen, interne Trennmittel (IMR), Füllstoffe, Farbstoffe, Pigmente, Flammschutzmittel, Hydrolyseschutzmittel, fungistatische und bakteriostatisch wirkende Substanzen sowie UV-Stabilisatoren und Antioxidantien. Möglich ist auch der Einsatz von Pigmenten und/oder Farbstoffen, um getönte/farbige Formkörper zu erhalten.

Die Mitverwendung eines Löse- oder Verdünnungsmittels für die Umsetzung gemäß Schritt (3) ist in der Regel nicht erforderlich. Im Rahmen einer bevorzugten Ausführungsform wird jedoch ein Lösemittel oder ein Gemisch aus zwei oder mehr Lösemitteln eingesetzt. Geeignete Lösemittel sind beispielsweise Kohlenwasserstoffe, insbesondere Toluol, Xylol oder Cyclohexan, Ester, insbesondere Ethylglykolacetat, Ethylacetat oder Butylacetat, Amide, insbesondere Dimethylformamid oder N-Methylpyrrolidon, Sulfoxide, insbesondere Dimethylsulfoxid, Ether, insbesondere Diisopropylether oder Methyl-tert.-butylether oder bevorzugt cyclische Ether, insbesondere Tetrahydrofuran oder Dioxan.

Darüber hinaus betrifft die vorliegende Erfindung auch ein Polyurethan mit einem niedrigen Gehalt an Verunreinigungen, erhältlich durch ein integriertes Verfahren aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxids zu einem Polyetheralkohol unter Verwendung mindestens einer Multimetallcyanidverbindung als Katalysator;
(3) Umsetzung eines Polyetheralkohols aus Schritt (2) mit mindestens einem Isocyanat.

Erfindungsgemäße Polyurethane zeichnen sich insbesondere durch einen niedrigen Gehalt an Verunreinigungen wie beispielsweise C6-Verbindungen aus. Damit sind die erfindungsgemäßen Polyurethane besonders für die Herstellung von Polyurethan-Schaum, Polyurethangießhäuten und Elastomeren geeignet.

Unter Polyurethan-Schaumstoffen werden insbesondere Schaumstoffe bevorzugt, die in der Automobil- und Möbelindustrie verwendet werden, wie HalbhartSchaumstoffe, Hartintegral- und Weichintegralschaumstoffe oder RIM-Werkstoffe (RIM = ReactionInjectionMouldiung).

Daher betrifft die vorliegende Erfindung auch einen Polyurethan-Schaum, erhältlich durch ein integriertes Verfahren aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxid zu einem Polyetheralkohol unter Verwendung mindestens einer Multimetallcyanid-Verbindung als Katalysator;
(3) Umsetzung eines Polyetheralkohols aus Schritt (2) mit mindestens einem Isocyanat;
(4) Verschäumen des durch die Umsetzung gemäß Schritt (3) erhaltenen Polyurethans.

Verfahren zur Herstellung von Polyurethan-Schäumen sind beispielsweise beschrieben in Becker, Braun, "Polyurethane", Kunststoffhandbuch Bd. 7, Carl-Hanser-Verlag, München, 3. Auflage, 1993, Seite 193 bis 265.

In einer bevorzugten Ausführungsform betrifft die vorliegende Erfindung ein Polyurethan, wobei das zur Herstellung des Polyurethans eingesetzte Polyetheralkohol erhältlich gemäß Schritt (2) mindestens einen Mischblock aus Ethylenoxid-Propylenoxid-Einheiten aufweist.

Ebenso betrifft die Erfindung ein Polyurethan, wobei der zur Herstellung des Polyurethans eingesetzte Polyetheralkohol erhältlich gemäß Schritt (2) mindestens einen terminalen Propylenoxid-Block aufweist.

Derartige Polyurethane sind beispielsweise geeignet zur Herstellung von Formkörpern, insbesondere Formkörpern aus Polyurethan-Blockweichschaum. Vorteilhaft ist hier der geringe Gehalt an Verunreinigungen, da so keine störenden Gerüche auftreten, die aus dem Weichschaum-Formteil austreten können.

Die engeren Molekulargewichtsverteilungen aufgrund des geringen Gehaltes an monofunktionellen Nebenverbindungen führt außerdem zu einer besseren Verarbeitungsbreite beim Verschäumen.

Daher betrifft die vorliegende Erfindung in einer weiteren Ausführungsform auch Formkörper umfassend ein Polyurethan oder einen Polyurethan-Schaum herstellbar mittels eines erfindungsgemäßen integrierten Verfahrens, sowie die Verwendung eines erfindungsgemäß hergestellten Polyurethans oder Polyurethanschaums zur Herstellung von Formkörpern.

Erfindungsgemäße Formkörper sind beispielsweise Matratzen, Kissen, Formteile für den Automobil-Innenausbau oder Polstermöbel.

Im einzelnen sind als erfindungsgemäße Formkörper zu nennen:
- Weichschaumstoffe, insbesondere Matratzen, Formteile für den Automobilinnenausbau, wie zum Beispiel Autositze, schallabsorbierende Formkörper, wie zum Beispiel Bodenteppiche und/oder Polstermöbel, Schwämme, Kissen, Kopfkissen, Sitzmöbeln, Bürostuhlpolstern, Rückenlehnen, orthopädischen Produkten;
- thermoplastische Polyurethane, insbesondere für die Verwendung von Kabel, Schläuche, Tiermarken, Schienenunterlagen, Folien, Schuhsohlen und Zubehör, Rollenbandagen, Skispitzen;
- Kaltgießelastomere, insbesondere für die Verwendung von Ummantelung von Hebe- und Tragegurten, Gewebebeschichtung, Beschichtung von Transportbändern, Prallschutzelemente, Industrielle Kantenschoner, Zahnriemen, Siebe für abrasive Schüttgüter, Abstreifer und Scharleisten, Fördersteme und Walzen, Walzenbeschichtung, Bodenschutzplatten für schwere Baumaschinen, Gehäuseteile, Beschichtung von Entgratungstrommeln, Pumpenelemente und Pumpengehäuse, Außenrohrbeschichtungen, Behälterauskleidungen, Fahrzeugbodenmatten, Molche, Zyklone, Schwerlastrollen, Umlenkrollen, Leitrollen, Lenk- und Bockrollen, Führungsrollen, Spezialbeschichtungen von Förderbändern, Hydrolyse- und abriebfeste Beschichtungen von Rinnen, Beschichtungen von LKW-Ladeflächen, Stoßfänger, Kupplungsteile, Bojenbeschichtungen, Inline-Skater-Rollen, Spezialrollen, Hochbelastbare Pumpenelemente;
- Weichintegralschaumstoffe, insbesondere Lenkräder, Luftfilterdichtungen, Schaltknopf, Kabelumschäumung, Behälterummantelung, Armlehnen, Schuhsohlen aus Polyurethan;
- Polyurethanbeschichteungen, insbesondere für Bodenbeläge, Veredlungen von Materialien wie Holz, Leder, Blechen;
- Polyurethan-Gießhäute, insbesondere für die Verwendungen als Einlagen für Formteile wie Autoarmaturen, Autotürverkleidungen, LKW- und Autositze, Bodenmatten;
- Polyurethan-Hartschaumstoffe, insbesondere für die Verwendung als Wärmedämmaterial oder als Konstruktionsmaterial;
- Hartintegral-Schaumstoffe, insbesondere für die Verwendung als Bau- und Dekorelemente im Innen- und Außenbereich, komplexe Möbel, Autoinnenraumelemente, Skier und Snowboards sowie technische Funktionsteile;
- RIM-Schaumstoffe, insbesondere zur Herstellung von Fertigteilen für die Verwendung im Automobilsektor im Außenbereich, wie Front- und Heckschürzen, Seitenschweller und im Nutzfahrzeugsektor, wie großflächige Verkleidungen, Kotflügel, Radverbreiterungen;
- Thermoformschaumstoffe, insbesondere zur Herstellung von ultraleichten Verbundkonstruktionen, für die Verwendung im Fahrzeugbau als Dachverkleidungselement;
- Halbhartschaumstoffe, insbesondere für die Hinterschäumung von Folien, Häuten oder Leder und Faser verstärkte Trägerbauteile, Halbhartschaumstoffen zur Herstellung von Auto-Schiebehimmeln für Auto-Glasdächer oder -Türseitenteilen.

## Patentansprüche

1. Integriertes Verfahren zur Herstellung eines Polyurethans aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxid zu einem Polyetheralkohol unter Verwendung mindestens einer Multimetallcyanidverbindung als Katalysator;
(3) Umsetzung eines Polyetheralkohols aus Schritt (2) mit mindestens einem Isocyanat.

2. Integriertes Verfahren zur Herstellung eines Polyurethans nach Anspruch 1, **dadurch gekennzeichnet, dass** das in Schritt (1) eingesetzte Hydroperoxid Wasserstoffperoxid ist.

3. Integriertes Verfahren zur Herstellung eines Polyurethans nach Anspruch 1 oder 2 , **dadurch gekennzeichnet, dass** das in Schritt (1) erhaltenen Propylenoxid einen Gehalt an C6-Komponenten < 1 ppm aufweist.

4. Integriertes Verfahren zur Herstellung eines Polyurethans nach einem der vorherigen Ansprüche, **dadurch gekennzeichnet, dass** die Epoxidation gemäß Schritt (1) in Gegenwart eines titanhaltigen Zeolithkatalysators durchgeführt wird.

5. Integriertes Verfahren zur Herstellung eines Polyurethans nach einem der vorherigen Ansprüche, **dadurch gekennzeichnet, dass** die Multimetallcyanidverbindung Zink, Kobalt oder Eisen oder zwei davon enthält.

6. Polyurethan mit einem niedrigen Gehalt an Verunreinigungen, erhältlich durch ein integriertes Verfahren aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxid zu einem Polyetheralkohol unter Verwendung mindestens einer Multimetallcyanidverbindung als Katalysator;
(3) Umsetzung eines Polyetheralkohols aus Schritt (2) mit mindestens einem Isocyanat.

7. Polyurethan nach Anspruch 6, **dadurch gekennzeichnet, dass** der zur Herstellung des Polyurethans eingesetzte Polyetheralkohol erhältlich gemäß Schritt (2) mindestens einen Mischblock aus Ethylenoxid-Propylenoxid-Einheiten aufweist.

8. Polyurethan nach Anspruch 6 oder 7, **dadurch gekennzeichnet, dass** der zur Herstellung des Polyurethans eingesetzte Polyetheralkohol erhältlich gemäß Schritt (2) mindestens einen terminalen Propylenoxid-Block aufweist.

9. Polyurethan-Schaum, erhältlich durch ein integriertes Verfahren aufweisend mindestens die folgenden Schritte:
(1) Epoxidation von Propen mit mindestens einem Hydroperoxid zu Propylenoxid;
(2) Umsetzung des Propylenoxids aus Schritt (1) oder eines Gemisches aus dem Propylenoxid aus Schritt (1) und mindestens einem weiteren Alkylenoxid zu einem Polyetheralkohol unter Verwendung mindestens einer Multimetallcyanidverbindung als Katalysator;
(3) Umsetzung eines Polyetheralkohols aus Schritt (2) mit mindestens einem Isocyanat;
(4) Verschäumen des durch die Umsetzung gemäß Schritt (3) erhaltenen Polyurethans.

10. Formkörper umfassend ein Polyurethan herstellbar mittels eines integrierten Verfahrens gemäß einem der Ansprüche 1 bis 5 oder ein Polyurethan gemäß einem der Ansprüche 6 bis 8 oder einen Polyurethan-Schaum gemäß Anspruch 9.

## Claims

1. An integrated process for the preparation of a polyurethane, comprising at least the following steps:
(1) epoxidation of propene with at least one hydroperoxide to give propylene oxide;
(2) reaction of the propylene oxide from step (1) or a mixture of the propylene oxide from step (1) and at least one further alkylene oxide to give a polyether alcohol using at least one multimetal cyanide compound as a catalyst;
(3) reaction of a polyether alcohol from step (2) with at least one isocyanate.

2. The integrated process for the preparation of a polyurethane according to claim 1, wherein the hydroperoxide used in step (1) is hydrogen peroxide.

3. The integrated process for the preparation of a polyurethane according to claim 1 or 2, wherein the propylene oxide obtained in step (1) contains < 1 ppm of C6 components.

4. The integrated process for the preparation of a polyurethane according to any of the preceding claims, wherein the epoxidation according to step (1) is carried out in the presence of a titanium-containing zeolite catalyst.

5. The integrated process for the preparation of a polyurethane according to any of the preceding claims, wherein the multimetal cyanide compound contains zinc, cobalt or iron or two thereof.

6. A polyurethane having a low content of impurities, obtainable by an integrated process comprising at least the following steps:
(1) epoxidation of propene with at least one hydroperoxide to give propylene oxide;
(2) reaction of the propylene oxide from step (1) or a mixture of the propylene oxide from step (1) and at least one further alkylene oxide to give a polyether alcohol using at least one multimetal cyanide compound as a catalyst;
(3) reaction of a polyether alcohol from step (2) with at least one isocyanate.

7. The polyurethane according to claim 6, wherein the polyether alcohol used for the preparation of the polyurethane and obtainable according to step (2) has at least one mixed block of ethylene oxide/propylene oxide units.

8. The polyurethane according to claim 6 or 7, wherein the polyether alcohol used for the preparation of the polyurethane and obtainable according to step (2) has at least one terminal propylene oxide block.

9. A polyurethane foam obtainable by an integrated process comprising at least the following steps:
(1) epoxidation of propene with at least one hydroperoxide to give propylene oxide;
(2) reaction of the propylene oxide from step (1) or a mixture of the propylene oxide from step (1) and at least one further alkylene oxide to give a polyether alcohol using at least one multimetal cyanide compound as a catalyst;
(3) reaction of a polyether alcohol from step (2) with at least one isocyanate;
(4) foaming of the polyurethane obtained by the reaction according to step (3).

10. A molding comprising a polyurethane which can be prepared by means of an integrated process according to any of claims 1 to 5 or a polyurethane according to any of claims 6 to 8 or a polyurethane foam according to claim 9.

## Revendications

1. Procédé intégré pour la préparation d'un polyuréthanne, comprenant au moins les étapes suivantes :
(1) époxydation du propène en oxyde de propylène à l'aide d'au moins un hydroperoxyde ;
(2) réaction de l'oxyde de propylène provenant de l'étape (1) ou d'un mélange de l'oxyde de propylène provenant de l'étape (1) et d'au moins un autre oxyde d'alkylène, pour l'obtention d'un polyéther-alcool, avec utilisation d'au moins un composé de type cyanure multimétallique en tant que catalyseur ;
(3) réaction d'un polyéther-alcool provenant de l'étape (2) avec au moins un isocyanate.

2. Procédé intégré pour la préparation d'un polyuréthanne selon la revendication 1, **caractérisé en ce que** l'hydroperoxyde utilisé dans l'étape (1) est le peroxyde d'hydrogène.

3. Procédé intégré pour la préparation d'un polyuréthanne selon la revendication 1 ou 2, **caractérisé en ce que** l'oxyde de propylène obtenu dans l'étape (1) a une teneur en composants en C6 inférieure à 1 ppm.

4. Procédé intégré pour la préparation d'un polyuréthanne selon l'une quelconque des revendications précédentes, **caractérisé en ce que** l'époxydation selon l'étape (1) est effectuée en présence d'un catalyseur de type zéolithe contenant du titane.

5. Procédé intégré pour la préparation d'un polyuréthanne selon l'une quelconque des revendications précédentes, **caractérisé en ce que** le composé de type cyanure multimétallique contient du zinc, du cobalt ou du fer ou deux de ces métaux.

6. Polyuréthanne ayant une faible teneur en impuretés, pouvant être obtenu par un procédé intégré comprenant au moins les étapes suivantes :
(1) époxydation du propène en oxyde de propylène à l'aide d'au moins un hydroperoxyde ;
(2) réaction de l'oxyde de propylène provenant de l'étape (1) ou d'un mélange de l'oxyde de propylène provenant de l'étape (1) et d'au moins un autre oxyde d'alkylène, pour l'obtention d'un polyéther-alcool, avec utilisation d'au moins un composé de type cyanure multimétallique en tant que catalyseur ;
(3) réaction d'un polyéther-alcool provenant de l'étape (2) avec au moins un isocyanate.

7. Polyuréthanne selon la revendication 6, **caractérisé en ce que** le polyéther-alcool utilisé pour la préparation du polyuréthanne, pouvant être obtenu selon l'étape (2), comporte au moins une séquence mixte de motifs oxyde d'éthylène-oxyde de propylène.

8. Polyuréthanne selon la revendication 6 ou 7, **caractérisé en ce que** le polyéther-alcool utilisé pour la préparation du Polyuréthanne, pouvant être obtenu selon l'étape (2), comporte au moins une séquence oxyde de propylène terminale.

9. Mousse de polyuréthanne, pouvant être obtenue par un procédé intégré comprenant au moins les étapes suivantes :
(1) époxydation du propène en oxyde de propylène à l'aide d'au moins un hydroperoxyde ;
(2) réaction de l'oxyde de propylène provenant de l'étape (1) ou d'un mélange de l'oxyde de propylène provenant de l'étape (1) et d'au moins un autre oxyde d'alkylène, pour l'obtention d'un polyéther-alcool, avec utilisation d'au moins un composé de type cyanure multimétallique en tant que catalyseur ;
(3) réaction d'un polyéther-alcool provenant de l'étape (2) avec au moins un isocyanate ;
(4) transformation en mousse du polyuréthanne obtenu par la réaction selon l'étape (3).

10. Corps moulé comprenant un polyuréthanne pouvant être préparé par un procédé intégré selon l'une quelconque des revendications 1 à 5 ou un polyuréthanne selon l'une quelconque des revendications 6 à 8 ou d'une mousse de polyuréthanne selon la revendication 9.
